# EUROPÄISCHE PATENTSCHRIFT

(11) **EP 1 326 819 B1**
(45) Veröffentlichungstag und Bekanntmachung des Hinweises auf die Patenterteilung: **11.01.2006**
(21) Anmeldenummer: 01986668.0
(22) Anmeldetag: 11.10.2001
(51) Int. Cl.: C07C 29/90, C07C 31/22

(54) **FARBZAHLVERBESSERUNG VON MEHRWERTIGEN ALKOHOLEN DURCH HYDRIERUNG**
IMPROVEMENT OF A COLOUR INDEX OF MULTIVALENT ALCOHOLS BY HYDROGENATION
AMELIORATION DE L'INDICE DE COULEUR D'ALCOOLS POLYVALENTS PAR HYDROGENATION

(30) Priorität: 13.10.2000 DE 10050645
(43) Veröffentlichungstag der Anmeldung: 16.07.2003
(73) Patentinhaber: BASF Aktiengesellschaft, 67056 Ludwigshafen (DE)
(72) Erfinder: DERNBACH, Matthias, 69221 Dossenheim (DE); SIGWART, Christoph, 69198 Schriesheim (DE); HESSE, Michael, 67549 Worms (DE); MAAS, Steffen, 55270 Bubenheim (DE)
(86) Internationale Anmeldenummer: PCT/EP2001/011763
(87) Internationale Veröffentlichungsnummer: WO 2002/030858

(56) Entgegenhaltungen:
- EP-A- 0 601 571
- WO-A-98/28253
- CHEMICAL ABSTRACTS, vol. 55, no. 21, 16. Oktober 1961 (1961-10-16) Columbus, Ohio, US; abstract no. 20996h, Spalte 20996; XP002171005 -& NL 97 091 C (STAMICARBON) 15. Februar 1961 (1961-02-15)

## Beschreibung

Die vorliegende Erfindung bezieht sich auf ein Verfahren, bei dem mehrwertige Alkohole geringer Farbzahl durch Hydrierung erhalten werden.

Mehrwertige Alkohole werden in großem Maßstab durch Kondensation von Formaldehyd mit höheren, CH-aciden Aldehyden oder mit Wasser und Acrolein bzw. 2-Alkylacroleinen erhalten. Dabei unterscheidet man bei dieser Reaktion zwischen zwei prinzipiellen Durchführungsvarianten.

Zum einen ist dies das sogenannte Cannizzaro-Verfahren, das wiederum unterteilt wird in das anorganische und das organische Cannizzaro-Verfahren. Bei der anorganischen variante setzt man einen Überschuß an Formaldehyd mit dem entsprechenden Alkanal in Gegenwart von stöchiometrischen Mengen einer anorganischen Base wie NaOH oder Ca(OH)₂ um. Das in der ersten Stufe gebildete Dimethylolbutanal reagiert in der zweiten Stufe mit dem überschüssigen Formaldehyd in einer Disproportionierungsreaktion zu Trimethylolpropan und dem Formiat der entsprechenden Base, also etwa zu Natrium- oder Kalziumformiat. Der Anfall dieser Salze stellte einen Nachteil dar, da sie schwierig vom Reaktionsprodukt abzutrennen sind, und außerdem ein Äquivalent Formaldehyd verloren geht.

Bei dem organischen Cannizzaro-Verfahren wird anstelle einer anorganischen Base ein tertiäres Alkylamin eingesetzt. Damit lassen sich höhere Ausbeuten erzielen als mit einer anorganischen Base. Es fällt als unerwünschtes Nebenprodukt Trialkylammoniumformiat an. Somit geht auch hier ein Äquivalent des Formaldehyds verloren.

Die Nachteile des Cannizzaro-Verfahrens werden bei dem sogenannten Hydrierverfahren vermieden. Dabei bringt man Formaldehyd mit dem entsprechenden Aldehyd in Gegenwart von katalytischen Mengen eines Amins zur Reaktion. Damit wird erreicht, daß die Reaktion auf der Stufe des alkylolierten Aldehyds anhält. Nach Abtrennung des Formaldehyds wird das Reaktionsgemisch, das neben dem erwähnten alkylolierten Aldehyd noch geringe Mengen des entsprechenden mehrwertigen Alkohols und von Acetalen der gebildeten Alkohole enthalt, einer Hydrierung unterworfen, bei der der gewünschte mehrwertige Alkohol erhalten wird.

Ein besonders effektives Verfahren zur Herstellung von durch Kondensation von Aldehyden mit Formaldehyd erhältlichen Alkoholen wird dabei in der WO 98/28253 beschrieben. Hohe Ausbeuten, verbunden mit den Anfallen geringer Mengen an Kuppelprodukten, werden mit diesem Verfahren ermöglicht. Es wird dabei so verfahren, daß der höhere Aldehyd mit der 2- bis 8-fachen Menge Formaldehyd in Gegenwart eines tertiären Amins umgesetzt wird, und man das so erhaltene Reaktionsgemisch in zwei Lösungen auftrennt, wobei eine Lösung das erwähnte vollständig methylolierte Alkanal und die andere Lösung nicht umgesetztes Ausgangsprodukt aufweist. Diese letzte Lösung wird in die Reaktion zurückgeführt. Die Auftrennung erfolgt durch Destillation oder einfaches Abtrennen der wässrigen von der organischen Phase. Die das Produkt enthaltende Lösung wird einer katalytischen und/oder thermischen Behandlung unterworfen, um nicht-vollständig alkylolierte Alkanale in die gewünschten vollständig methylolierten Verbindungen zu überführen. Hierbei entstandenes Nebenprodukt wird durch Destillation abgetrennt, und der so erhaltene Sumpf wird der katalytischen Hydrierung, die zu den mehrwertigen Alkoholen führt, unterworfen.

Beispiele für wichtige, mit den beschriebenen Verfahren hergestellte Alkohole sind Neopentylgylkol, Pentaerythrit, Trimethylolethan, Trimethylolbutan und insbesondere Trimethylolpropan (TMP).

TMP hat ein breites Einsatzfeld als Vernetzer von Polyestern und von Polyurethanen gefunden. Kommerziell erhältliche TMP-Qualitäten weisen jedoch eine mehr oder weniger ausgeprägte Färbung auf, die vermutlich durch das Vorliegen von Verunreinigungen verursacht wird. Bei vielen Verwendungen ist diese Färbung nicht störend. Es gibt jedoch auch Anwendungen, in denen der Einsatz von möglichst farblosem TMP wünschenswert ist. In der Literatur sind verschiedene Verfahren beschrieben, mit denen eine Farbzahlverbesserung des TMP erreicht werden soll.

Die US 3,097,245 beschreibt ein Verfahren zur Herstellung von Trimethylolpropan mit einer APHA-Farbzahl zwischen 50 und 200. Diese Farbzahl wird dabei durch das Einhalten bestimmter Reaktionsbedingungen hinsichtlich Temperatur. Reaktionszeit, pH-Wert und Konzentration der Ausgangsverbindungen erreicht. Weiterhin schließt sich an die Reaktion die Behandlung der erhaltenen Lösung mit einem Ionenaustauscherharz an.

Die US 5,603,835 offenbart ein verfahren zur Herstellung von TMP mit APHA-Farbzahlen von < 100. Diese werden erreicht durch extraktive Nachbehandlung der erhaltenen rohen TMP-Lösungen mit einem Ether oder einem Ester. Die eingesetzten TMP-Losungen stammen im allgemeinen aus dem Cannizzaro-Verfahren.

Die beiden vorstehend beschriebenen Verfahren weisen den Nachteil auf, daß sie relativ aufwendig sind, da bestimmte Bedingungen genau eingehalten werden müssen und die Zugabe eines Ionenaustauscherharzes nötig ist, bzw, die Einführung mindestens eines Lösungsmittels notwendig wird.

Zur Hydrierung von bei der Kondensation von Formaldehyd mit höheren Aldehyden entstehenden Produkten finden sich in der Literatur nur wenige Informationen.

Die DE-A-17 68 259 offenbart ein Verfahren zur Verarbeitung der Nebenprodukte, die bei der Umsetzung von Formaldehyd mit höheren Aldehyden zu mehrwertigen Alkoholen entstehen. Das Verfahren besteht darin, diese Nebenprodukte nach der Abtrennung vom Hauptprodukt einer Hydrierung zu unterwerfen. Dabei werden in größeren Mengen hauptsächlich aliphatische Alkohole gewonnen.

In WO 99/20586 wird ein Verfahren zur Herstellung von Trimethylolpropan mit einer niedrigen "reacted color" (Farbzahl nach standardisierten Kochtests) beschrieben. Das durch Cannizzaro-Reaktion hergestellte Roh-Trimethylolpropan wird mit einem Wasser-Lösungsmittelgemisch erhitzt, so dass eine einphasige Lösung ensteht. Dann wird abgekühlt, wobei sich die Lösung in mindestens 2 Phasen separiert. Das Trimethylolpropan wird aus der wässrigen Phase durch eine zweistufige Destillation gewonnen und weist in der Regel eine "Acid wash color" von 1 bis 1,5 Gardner (GU) und eine Phtalanhydrid-Farbzahl von 1 GU auf. Nachteilig bei diesem Verfahren ist der Verlust von mehr als 10% Trimethylolpropan, das sich in der organischen Phase löst. Das verfahren ist zudem technisch aufwendig und eine Verbesserung der natürlichen Farbzahl nicht berichtet.

Das in der SU-A 125 552 beschriebene Hydrierverfahren dient der Reinigung von nach dem Cannizzaro-Verfahren erhaltenem TMP. Es wird dabei entweder rohes TMP in Form einer wässrigen Lösung enthaltend ca. 30 % TMP, oder ein gereinigtes, von Wasser und Formiaten befreites TMP mit einem Gehalt von ca. 80 % an TMP eingesetzt. Man erhält durch Hydrierung an Nickel-, Zink-, Molybdän- und Kupferkatalysatoren reines TMP mit einem Gehalt von ca. 98 % nach Destillation. Die verwendeten Drücke betragen 1 bis 250 bar, vorzugsweise 10 bis 200 bar, die Temperaturen sind 20 bis 200°C, vorzugsweise 100 bis 150°C. Es wird erwähnt, daß das erhaltene TMP farblos ist, wobei jedoch keine Farbsahl angegeben wird.

Es hat sich jedoch gezeigt, daß die mit diesem Verfahren erhältlichen Farbzahlverbesserungen für viele Zwecke oft nicht ausreichend sind.

Die der vorliegenden Erfindung zugrundeliegende Aufgabe ist daher die Bereitstellung eines Verfahrens, das es erlaubt, mehrwertige Alkohole, insbesondere TMP, mit geringer Farbzahl zu erhalten. Dabei sollte es möglich sein, APHA-Farbzahlen von < 20 zu erreichen.

Diese Aufgabe wird gelöst durch ein Verfahren zur Farbzahlverbesserung von mehrwertigen Alkoholen durch katalytische Hydrierung, wobei in der Hydrierung ein mehrwertiger Alkohol eingesetzt wird, der nach seiner Herstellung durch Destillation gereinigt wurde, dadurch gekennzeichnet, dass in Gegenwart eines makroporösen, geträgerten Heterogenkatalysator, der als Aktivmetall mindestens ein Metall der VII. bis X. Nebengruppe des Periodensystems enthält, hydriert wird.

Es wurde festgestellt, daß durch den Einsatz von bereits destilliertem mehrwertigem Alkohol und Durchführung der Hydrierung in Gegenwart eines makroporösen, geträgerten Heterogenkatalysator, der als Aktivmetall mindestens ein Metall der VII. bis X. Nebengruppe des Periodensystems enthält, eine Farbzahlverbesserung erreicht werden kann, die weitaus größer ist als bei einem nicht destillativ vorgereinigten Alkohol. Gute Ergebnisse wurden dabei mit Lösungen erhalten, die einen Alkohol-Gehalt von > 95 % aufweisen.

Wenn TMP in die Hydrierung eingesetzt wird, können besonders gute Ergebnisse beim Einsatz von TMP-Lösungen mit einem Gehalt von > 98 % erzielt werden.

Das erfindungsgemäße Verfahren kann dabei zur Farbzahlverbesserung von mehrwertigen Alkoholen, insbesondere TMP, jeglicher Herkunft verwendet werden. Chargen, die dem organischen oder dem anorganischen Cannizzarro-Verfahren entstammen, können ebenso in der Farbzahlverbesserungshydrierung nach der vorliegenden Erfindung eingesetzt werden wie aus dem Hydrierverfahren stammende Alkohole, bevorzugt werden jedoch aus dem Hydrierverfahren stammende Alkohole eingesetzt. Es ist lediglich wichtig, daß der Alkohol vorher gereinigt wurde und eine Reinheit aufweist, die in dem oben genannten Bereich liegt und somit eine Verbesserung der Farbzahl mittels des erfindungsgemäßen Verfahrens gestattet.

Die erfindungsgemäße Hydrierung ist auf alle mehrwertigen Alkohole anwendbar, die durch Kondensation von Formaldehyd mit höheren Aldehyden unter Zugabe katalytischer Mengen Trialkylamin und nachfolgender Hydrierung hergestell werden können. Geeignete höhere Aldehyde sind praktisch alle Alkanale mit einem aciden Wasserstoifatom in α-Stellung zur Carbonylgruppe. Es können aliphatische Aldehyde mit 2 bis 24 C-Atomen als Ausgangsmaterialien verwendet werden, die geradkettig oder verzweigt sein oder auch alicyclische Gruppen enthalten können. Ebenso sind araliphatische Aldehyde als Ausgangsstoffe geeignet, vorausgesetzt daß sie eine Methylengruppen in α-Stellung zur Carbonylgruppe enthalten. Im allgemeinen werden Aralkylaldehyde mit 8 bis 24 C-Atomen, vorzugsweise mit 8 bis 12 C-Atomen als Ausgangsmaterialien verwendet, beispielsweise Phenylacetaldehyd. Bevorzugt werden aliphatische Aldehyde mit 2 bis 12 C-Atomen, beispielsweise 3-Ethyl-, 3-n-Propyl-, 3-Isopropyl-, 3-n-Butyl-, 3-Isobutyl-, 3-sek.-Butyl-, 3-tert.-Butyl-butanal sowie entsprechende n-Pentanale, -n-hexanale,-n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, -n-hexanale, -n-heptanale; 5-Ethyl-, 5-n-Propyl-, 5-Isopropyl-, 5-n-Butyl-, 5-Isobutyl-, 5-sek.-Butyl-, 5-tert.-Butyl-n-hexanale, -n-heptanale; 3-Methyl-hexanal, 3-Methyl-heptanal; 4-Methyl-pentanal,
4-Methyl-heptanal, 5-Methyl-hexanal, 5-Methylheptanal; 3,3,5-Trimethyl-n-pentyl-, 3,3-Diethylpentyl-,
4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl, 3,4-Dimetrylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diethylhexyl-, 3-Methyl-4-ethylpentyl, 3-Methyl-4-ethylhexyl-, 3,3,4-Trimethylpentyl-, 3,4,4-Trimethylpentyl-3,3,4-Trimethylhexyl-,
3,4,4-Trimethylhexyl-, 3,3,4,4,-Tetramethylpentylaldehyd; insbesondere C₂- bis C₁₂-n-Alkanale.

Besonders bevorzugte mehrwertige Alkohole im Rahmen der vorliegenden Erfindung sind Trimethylolethan, Trimethylolpropan, Trimethylolbutan, Neopentylglykol und Pentaerythrit. Der meisz bevorzugte Alkohol ist Trimethylolpropan.

Soll mit dem erfindungsgemäßen Verfahren die Farbzahl von TMP verbessert werden, kann ein nach dem Hydrierverfahren hergestelltes TMP hoher Reinheit (> 98 %) beispielsweise erhalten werden nach dem in der deutschen Anmeldung DE 19963435.1 mit dem Titel "Verfahren zur Reinigung von durch Hydrierung hergestelltem Trimethylolpropan durch kontinuierliche Destillation" (Anmelder: BASF AG) beschriebenen Verfahren. Dabei wird das nach Hydrierung erhaltene Rohprodukt zunächst einer Entwässerung unterzogen, bei der Wasser und andere Leichtsieder wie Methanol, Trialkylamin oder Trialkylammoniumformiat durch Destillation abgetrennt werden. Diese Destillation kann dabei bei Drücken< 400 mbar, vorzugsweise 20 bis 200 mbar, Sumpf temperaturen < 200°C und kurzen Verweilzeiten so durchgeführt werden, daß das gebildete Trialkylammoniumformiat mit TMP nur in geringem Maß zu TMP-Formiaten und Trialkylamin abreagiert. Es ist auch möglich, die Destillation bei Drück von > 200 mbar, vorzugsweise > 400 mbar, Sumpftemperaturen von > 140°C und kurzen Verweilzeiten so durchzuführen, daß zumindest der überwiegende Teil des TMP mit Trialkylammoniumformiat zu TMP-Formiaten und Trialkylamin reagiert.

Anschließend werden im nächsten Schritt die Hochsieder abgetrennt. Dies geschieht dadurch, daß vom Sumpf bei 210 bis 250°C diejenigen Komponenten durch Destillation abgetrennt werden, die bei diesen Temperaturen flüchtig sind. Die Hochsieder verbleiben somit im Sumpf. Die erhaltene, leichtsiedende TMP-reiche Fraktion wird anschließend destillativ aufgearbeitet (erste Reindestillation), wobei unerwünschte Leichtsieder abgetrennt werden. Das erhaltene Reinprodukt kann einer zweiten Reindestillation unterworfen werden, um ein besonders sauberes Produkt zu erhalten.

Das in dieser Anmeldung beschriebene verfahren kann auch noch weiter variiert werden. So ist es beispielsweise möglich, gebildetes TMP-Formiat mit einem geeigneten Amin, vorzugsweise einem Dialkylamin, zu TMP und Dialkylformamid umzusetzen. Ein solches Verfahren ist in der deutschen Anmeldung DE 19963444.0 mit dem Titel "Verfahren zur Umwandlung von bei der Trimethylolalkan-Herstellung gebildeten Trimethylolalkanformiat" (Anmelderin: BASF AG) beschrieben.

Weiterhin kann auch eine Steigerung der Ausbeuce durch Zersetzung der Hochsieder mittels Säurezugabe zu TMP und anderen Produkten erzielt werden. Die Beschreibung eines derartigen Verfahrens findet sich in der deutschen Anmeldung DE 19963437.8 mit dem Tizel "Verfahren zur Zersetzung von bei der Synthese mehrwertiger Alkohole gebildeter Nebenprodukte" (Anmelderin: BASF AG).

Es konnten gute Ergebnisse mit einem derart oder auf eine andere Weise destillativ gereinigten TMP, das APHA-Farbzahlen von 1 bis 500 APHA, vorzugsweise 15 bis 120 APHA aufweist, erhalten werden.

Durch die Hydrierung nach der vorliegenden Erfindung lassen sich mehrwertige Alkohole mit APHA-Farbzahlen < 10 herstellen. Insbesondere läßt sich TMP mit einer Farbzahl von 1 APHA erhalten.

Die erfindungsgemäße Hydrierung wird bei Temperaturen von 20 bis 300°C, vorzugsweise 100 bis 180°C durchgeführt, wobei Drücke von 1 bis 350 bar, vorzugsweise 1 bis 100 bar, Anwendung finden. Die dabei benutzten Verweilzeiten betragen 5 Minuten bis 20 Stunden, vorzugsweise 10 Minuten bis 8 Stunden. Es kann dabei eine diskontinuierliche, sogenannte Batch-Fahrweise gewählt werden, die vorzugsweise im Rührkessel durchgeführt wird. Ebenso gut ist es mbolich, die Hydrierung kontinuierlich durchzuführen, vorzugsweise in Rohrreaktoren in der Sumpf- oder Rieselfahrweise.

Als makroporöse Hydrierungkatalysatoren finden in dem erfindungsgemäßen Verfahren heterogene makropröse, geträgerte Katalysatoren Verwendung, die als Aktivmetall mindestens ein Metall der VII. bis X. Der makroporöse Heterogenkatalysator kann als Aktivmetall prinzipiell alle Metalle der VII. bis X. Nebengruppe des Periodensystems der Elemente enthalten. Vorzugsweise werden als Aktivmetalle Palladium, Ruthenium, Rhenium, Nickel, Eisen und Cobalt oder ein Gemisch aus zwei oder mehreren Aktivmetallen eingesetzt, wobei insbesondere Palladium als Aktivmetall verwendet wird.

Der Begriff "makroporös" wird im Rahmen der vorliegenden Erfindung so verwendet, wie er in Pure Appl. Chem., 46, S 79 (1976) definiert ist, nämlich als Poren, deren Durchmesser oberhalb von 50 nm liegen. Der Gehalt an Makroporen des erfindungsgemäß verwendeten Heterogenkatalysators mit einem Porendurchmesser von mehr als 100nm, bezogen auf die Gesamtporen, ä.h die Makroporosität des Heterogenkatalysators liegt bei mehr als 10 Vol.-%, bevorzugt bei mehr als 20 Vol.-%, besonders bevorzugt bei 25 bis 90 vol.-% jeweils bezogen auf die Gesamtporen.

Der Gehalt des Aktivmetalls beträgt im allgemeinen 0,01 bis 10 Gew.-%, vorzugsweise 0,05 bis 5 Gew.-% und insbesondere 0,1 bis 3 Gew.-% bezogen auf das Gesamtgewicht des Katalysators.

Die Metalloberfläche auf dem erfindungsgemäß verwendeten makropösen Trägerkatalysator beträgt dabei insgesamt vorzugsweise 0,01 bis 10 m²/g, weiter bevorzugt ungefähr 0,05 bis 5 m²/g, insbesondere ungefähr 0,05 bis 3 m²/g des Katalysators. Die Metalloberfläche wird mittels von J. LeMaitre et al. in "Characterization of Heterogeneous Catalysts", Hrsg.Francis Delanney, D, New York 1984, S. 310-324, beschriebenen Chemisorptionsverfahren bestimmt.

Die erfindungsgemäß verwendeten makroporösen Heterogenkatalysatoren können technisch durch verschiedene an sich bekannte Verfahren, zum Beispiel durch Auftragen mindestens eines Metalls der VII. bis X. Nebengruppe des Periodensystems der Elemente auf einen geeigneten makroporösen Träger hergestellt werden.

Die Auftragung kann durch Tränken des Trägers mit wäßrigen Metallsalzlösungen auf den Träger, wie z. B. wäßrige Palladiumsalzlösungen, durch Aufsprühen entsprechender Metallsalzlösungen auf den Träger oder durch andere geeignete Verfahren erreicht werden. Als Metallsalze der VII. bis X. Nebengruppe des Periodensystems der Elemente eignen sich Nitrate, Nitrosylnitrate, Halogenide, Carbonate, Carboxylate, Acetylacetonate, Chlorokomplexe, Nitrokomplexe oder Aminkomplexe der entsprechenden Metalle, wobei Nitrate und Nitrosylnitrate bevorzugt sind. Bei Katalysatoren die mehrere Metalle der VII. bis X. Nebengruppe des Periodensystems enthalten, können die Metallsalze bzw. deren Lösungen gleichzeitig oder nacheinander aufgebracht werden.

Die mit Metallsalzlösungen beschichteten bzw. getränkten Träger werden anschließend, vorzugsweise bei Temperaturen zwischen 100 °C und 150°C getrocknet und wahlweise bei Temperaturen zwischen 200°C und 600°C, vorzugsweise 350°C bis 450°C, calciniert. Bei getrennten Auftränkung wird der Katalysator nach jedem Tränkschritt getrocknet und wahlweise caiciniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung im Gasstrom, der freien Wasserstoff enthält, bei Temperaturen von 30°C bis 600°C, vorzugsweise zwischen 150°C und 450°C aktiviert. Vorzugsweise besteht der Gasstrom aus 50 bis 100 Vol.-% H₂ und 0 bis 50 Vol.-% N₂.

Die zur Herstellung der erfindungsgemäß verwendeten Katalysatoren verwendbaren Trägermaterialien sind solche, die makroporös sind und einen mittleren Porendurchmesser von mindestens 50 nm, vorzugsweise mindestens 100 nm , insbesondere 500 nm, aufweisen und deren Oberfläche nach BET höchstens ungefähr 300 m²/g, vorzugsweise ungefähr 15 m²/g, weiter bevorzugt ungefähr 10 m²/g, insbesondere ungefähr 5 m²/g und weiter bevorzugt höchstens 3 m²/g aufweist.

Die Oberfläche des Trägers wird bestimmt nach dem BET-Verfahren durch N₂-Adsorption, insbesondere nach DIN 66131. Die Bestimmung von Porendurchmesser und Porenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Makroporosität aufweisen, eingesetzt werden können, werden vorzugsweise Aktivkohle, Siliciumcarbid, Siliciumoxid, Mullit, Cordierit, Aluminiumoxid, Titanoxid, Zirkoniumoxid, Magnesiumoxid, Zinkoxyd oder deren Gemische, weiter bevorzugt Aluminiumoxid und Zirkoniumdioxid und Mullit, eingesetzt, besonders bevorzugt Aluminiumoxid.

Weiterhin können die erfindungsgemäß verwendeten makroporösen Heterogenkatalysatoren technisch nach dem aus der EP-A- 653 243 bekannten Verfahren hergestellt werden, bei dem Aktivmetall und Makroporen in einem Schritt eingebracht werden. Nach dem aus der EP-A- 653 243 bekannten Verfahren werden die erfindungsgemäß verwendeten makroporösen Heterogenkatalysatoren durch Lösen eines wasserlöslichen Salzes eines Metalls der VII. bis X. Nebengruppe des Periodensystems der Elemente in einem organischen Lösungsmittel, Versetzen der so erhaltenen Lösung mit einem organischen Polymeren, das in der Lage ist, mindestens das Zehnfache seines Eigengewichts an Wasser zu binden, anschließendem Vermischen des Polymeren mit einem Katalysatorträgermaterial und Formen der so erhaltenen Masse, Trocknen und Calcinieren hergestellt.

Als wasserlösliche Salze eines Metalls der VII. bis X. Nebengruppe des Periodensystems der Elemente eignen sich bevorzugt Nitrate, Nitosylnitrate, Halogenide, Carbonate, Carboxylate, Acetylacetonate, Chlorokomplexe, Nitrokomplexe oder Aminkomplexe der entsprechenden Metalle, wobei Nitrate und Nitrosylnitrate besonders bevorzugt sind. Bevorzugte Lösungsmittel sind mit Wasser mischbare Lösungsmittel wie Alkohole, Ether und Amine. Als Alkohole sind insbesondere C1-C4-Alkohole wie Methanol, Ethanol, isoPropanol und n-Butanol zu nennen; als Ether kommt z.B Tetrahydrofuran in Betracht. Geeignete Amine sind beispielsweise Ammoniak, Monoamine wie Diethylamin, Methylamin, Triethylamin, Ethylamain, Propylamin und Butylamin.

Als organisches Polymer werden bevorzugt vernetzte Polymere aus Acrylsäure, Acrylsäure und Acrylamid sowie aus Acrylamid eingesetzt, wobei teilweise neutralisierte Natriumpolyacrylate, die schwach vernetzt sind, besonders bevorzugt sind. Als chemische Vernetzer kommen beispielsweise Diole wie Ethylenglykol, Polyethylenglykol und Polyole, Diamine, Diene in Mengen von 0,1 bis 5 Gew.-%, bezogen auf das Polymer, in Frage.

Bevorzugte Trägermaterialien sind Aktivkohle, Siliciumcarbid, Aluminiumoxid, Titanoxid, Mullit, Cordierit, Zirkoniumoxid, Magnesiumoxid, Zinkoxyd oder deren Gemische, weiter bevorzugt Aluminiumoxid und Zirkoniumdioxid und Mullit, besonders bevorzugt Aluminiumoxid.

Weitere Details zur Herstellung der erfindungsgemäß verwendeten makroporösen Katalysatoren unter Einbringung von Aktivmetall und Makroporen in einem Schritt sind der EP-A- 653 243 zu entnehmen.

Weiterhin können die erfindungsgemäß verwendeten makroporösen Katalysatoren unter Verwendung von Porenbildnern nach dem aus der EP-A 842 699 bekannten Verfahren technisch hergestellt werden. Als Porenbildner können dabei alle wassermischbaren Polymere eingesetzt werden, sofern sie eine Molmasse von mehr als ungefähr 6.000 bis 500.000 g/mol aufweisen. Ihr Molekulargewicht beträgt vorzugsweise ungefähr 10.000 bis ungefähr 200.000 g/mol, weiter bevorzugt ungefähr 13.000 bis ungefähr 150.000 g/Mol und insbesondere ungefähr 13.000 bis ungefähr 50.000 g/mol. Beispiele für verwendbare Polymere schließen Polyvinylchlorid, Copolymere eines Olefins mit polaren Comonomeren, wie z.B. Ethylen oder Propylen mit Polyvinylchlorid, Polyvinylidenchloridcopolymere, ABS-Harze, Polyethylencopolymere mit Vinylacetat, Alkylacrylate, Acrylsäure, chlorierte Polyethylene, chorsulfonoierte Polyethylene, thermoplastische Polyurethane, Polyamide wie z.B Nylon-5, Nylon-12, Nylon-6,6, Nylon-6,10, Nylon-11, Fluor enthaltende Harze wie z.B. FEP, Polyvinylidenfluorid, Polychlortrifluorethylen, Acrylnitril-Meth(methyl)acrylat-Copolymere, wie z.B. Methacrylnitril-Styrol-Copolmere, Polyalkyl(meth)acrylate, Celluloseacetat, Celluloseaceatbutyrst, Polycarbonate, Polysulfone, Polyphenyloxid, Polyester, wie z.B. Polybutylenterephthalat und polyvinylalkohol ein, wobei Polyvinylalkohol besonders bevorzugt ist.

Für die Herstellung der erfindungsgemäß verwendeten makroprösen Katalysatoren wird zunächst eine Aluminiumlegierung aus Aluminium und dem Aktivmetall der VII. bis X. Nebengruppe des Periodensystems der Elemente in bekannter weise gemäß dem aus der DE 2 159 736 bekannten Verfahren hergestellt.

Gemäß EP-A-0 842 699 erfolgt dann die Herstellung einer Knetmasse aus dieser Legierung, einem Verformungsmittel, Wasser und dem Porenbildner, Verformen dieser Knetmasse zu Formkörpern, Calcinieren des Formkörpers und schließlich das Behandeln des calcinierten Formkörpers mit einem Alkalimetallhydroxid.

Weitere Details dieses technischen Herstellungsverfahrens für die erfindungsgemäß verwendeten makroporösen Katalysatoren sind der EP-A- 842 699 zu entnehmen.

Bevorzugt werden die erfindungsgemäß verwendeten makroporösen Katalysatoren durch Auftränken mindestens eines Metalls der VII. bis X. Nebengruppe des Periodensystems der Elemente auf einen geeigneten makroporösen Träger hergestellt.

Gewünschtenfalls können die erfindungsgemäß verwendeten makroporösen Katalysatoren zusätzlich durch Basen dotiert werden, um eine Rückspaltung des Polymers während der Hydrierung vor allem bei hohen Hydriertemperaturen zu vermeiden. Geeignete Basen sind beispielsweise basische Oxide wie Alkali- oder Erdalkalimetalloxide wie beispielsweise Natriumoxiäe, Kaliumoxide, Calciumoxide, Bariumoxide. Besonders bevorzugt sind Natriumoxide. Diese Oxide, bzw. ihre Precursor wie die jeweiligen Hyäroxide, Carbonate oder Hydroxidcarbonate, können beispielsweise durch Tränkung in überstehender Lösung, Sprühimprägnierung oder während der Aufbauagglomeration des Trägers in Konzentrationen von 0,05 - 5 %, bezogen auf das Gewicht des Katalysators, auf den Katalysator aufgebracht werden. Gegebenenfalls schliesst sich eine Temperung zur thermischen Zersetzung der Precursor an.

Die erfindungsgemäß verwendeten makroporösen Katalysatoren können vor Ihrem Einsatz in der Hydrierung mit Wasserstoff vorreduziert werden.

Die erfindungsgemäß anwendbaren makroporösen Katalysatoren können in Form von Pulver, beispielsweise bei der Durchführung des Verfahrens in Suspensionsfahrweise, oder zweckmäßigerweise als Formkörper, z.B. in Form von Strängen, Zylindern, Kugeln, Ringen oder Splitt, insbesondere bei einer Festbettanordnung des Katalysators, im erfindungsgemäßen Verfahren eingesetzt werden.

Die erfindungsgemäße Hydrierung unter Verwendung der oben be-schriebenen makroporösen Heterogenkatalysatoren wird vorzugsweise im Festbett durchgeführt. Dabei hat es sich als vorteilhaft erwiesen, die Reaktion in einem Hauptreaktor im geraden Durchgang durchzuführen. Genauso gute Resultate wurden erhalten, wenn die Hydrierung in einem Hauptreaktor mit nachgeschaltetem Nachreaktor durchgeführt wurde, wobei der Hauptreaktor in Kreislauffahrweise und der Nachreaktor im geraden Durchgang betrieben wurde. Es kann dabei jeweils die Sumpf- oder Rieselfahrweise gewählt werden. Bevorzugt ist Festbett im geraden Durchgang in Sumpffahrweise.

Die erfindungsgemäße Hydrierung des destillativ vorgereinigten TMP kann mit oder ohne Zugabe eines weiteren Lösungsmittels erfolgen. Wird ein solches Lösungsmittel verwendet, so wird dieses in solchen Konzentrationen zugegeben, daß die in die Hydrierung eingesetzten Lösungen einen TMP-Gehalt von 5 bis 95 Gew.-% aufweisen. Als Lösungsmittel werden dabei bevorzugt leichtsiedende organische Lösungsmittel wie Alkohole, Ether, Kohlenwasserstoffe, oder Ester eingesetzt. Bevorzugte Lösungsmittel umfassen Methanol, Ethanol, n-Propanol, i-Propanol, Butanol, Diethylether, Methyl-tert-Butylether, Dioxan, Tetrahydrofuran und Essigsäureethylester. Besonders bevorzugte Lösungsmittel sind Methanol und Tetrahydrofuran. Wird die erfindungsgemäße Hydrierung in Gegenwart eines zusätzlichen Lösungsmittels durchgeführt, so wird hinter die Hydrierung eine Trenneinheit geschaltet, um das Lösungsmittel von dem derart erhaltenen TMP abzutrennen. Übliche Trennein-heiten sind beispielsweise Destillationskolonnen, Dünnschichtverdampfer und vorzugsweise Fallfilmverdampfer.

Das erfindungsgemäße verfahren wird nun anhand der nachfolgenden Beispiele erläutert. Dabei wurde in allen Beispielen TMP verwendet, das wie folgt hergestellt worden war:

Eine Apparatur bestehend aus zwei beheizbaren, durch Überlaufrohre miteinander verbundenen Rührkesseln mit einem Fassungsvermögen von insgesamt 72 l wurde mit frischer, wäßriger Formaldehydlösung (4300g/h in Form der 40 %igen wäßrigen Lösung und n-Butyraldehyd (1800 g/h) und mit frischem Trimethylamin als Ka-talysator (130 g/h) in Form der 45 %igen wäßrigen Lösung kontinuierlich beschickt. Der Reaktoren wurden dabei auf 40°C temperiert.

Der Austrag wurde direkt in den oberen Teil eines Fallfilmver-dampfers mit aufgesetzter Kolonne geleitet und dort bei normalem Druck destillativ in ein leichtsiedendes Kopfprodukt, im wesentlichen erhaltend n-Butyraldehyd, Ethylacrolein, Formaldehyd, Wasser und Trimethylamin, und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33,5 kg/h) wurde kontinuierlich mit frischem Trimethylamin-Katalysator (50 g/h, in Form der 45 %igen wäßrigen Lösung) versetzt und in einen beheizbaren, mit Füllkörpern versehenen Rohrreaktor mit einem Leervolumen von 12 l geführt. Der Reaktor war dabei auf 40°C temperiert.

Der Austrag des Nachreaktors wurde kontinuierlich in den oberen Teil einer weiteren Destillationseinrichtung, der Formaldehydabtrennung (11 bar Heizdampf), gegeben und dort destillativ in ein leichtsiedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltene Sumpfprodukt enthielt neben Wasser im wesentlichen Dimethylolbutyraldehyd, Formaldehyd und Spuren Monomethylolbutyraldehyd. Er wurde dann einer kontinuierlichen Hydrierung unterworfen. Dazu wurde die Reaktionslösung bei 90 bar und 115°C in einem Hauptreaktor in Kreislauf/Rieselfahrweise und einem nachgeschalteten Nachreaktor in Kreislauffahrweise hydriert. Der Katalysator wurde analog DE 19809418, Beispiel J hergestellt und enthielt 42 Gew% CuO, 16 Gew% Cu und 46% TiO2. Die verwendete Apparatur bestand aus einem 10 m langen beheizten Hauptreaktor (Innendurchmesser: 27 mm) und einem 5,3 m langen beheizten Nachreaktor (Innendurchmesser: 25 mm). Der Kreislauf durchsatz betrug 25 l/h Flüssigkeit, der Reaktorzulauf wurde auf 4 kg/h eingestellt. Dementsprechend wurden 4 kg/h Hydrieraustrag erhalten.

Das TMP wurde nach der Hydrierung dem Sumpf entnommen und entsprechend der in Beispielen 2 und 3 der deutschen Anmeldung DE 19963435.1 mit dem Titel "Verfahren zur Reinigung von durch Hydrierung hergestelltem Trimethylolpropan durch kontinuierliche Destillation (Anmelderin: BASF AG) beschriebenen Methode destillativ aufgearbeitet. In einigen Beispielen wurde dabei TMP verwendet, das der ersten Reindestillationskolonne entnommen wurde (Qualität A). In anderen Beispielen wurde wiederum TMP verwendet, das der zweiten Reindestillation entstammte (Qualität B).

Das bereits mit dem erfindigsgemäßen Verfahren hydrierte TMP kann einer weiteren Hydrierung zur verbesserung der Farbzahl unterworfen werden, wobei vorzugsweise ein anderer Katalysator verwendet wird.

Die angegebenen APHA-Farbzahlen wurden mit einem LICO 200-Gerät der Firma Dr. Lange gemessen. Die TMP-Proben wurden dabei in reiner Form in Schmelze bei 100°C gemessen.

### Beispiele

### Herstellung Katalysator A

119 g einer wässrigen Palladiumnitratlösung (12,6 Gew.-% Palladium) wurden mit 1868 ml Wasser verdünnt und auf 5985 g eines makroporösen Aluminiumträger in Strangform (4 mm Stränge, alpha-Al₂O₃, BET-Oberfläche 6,5 m²/g) aufgesprüht. Die Trocknung erfolgte bei 120°C in ruhender Schicht. Anschließend wurde 2 h bei 500 °c getempert. Der Katalysator A enthielt 0,21 Gew.-% Palladium und einen Makroporenanteil von ungefähr 61 %.

### Beispiel 1

Es wurde TMP der Qualität A hydriert, das eine Farbzahl von 105 APHA und einen TMP-Gehalt von 99,0 % aufwies. Die Hydrierung erfolgte dabei in einem Rohrreaktor in Sumpffahrweise an 100 ml des Katalysators A. Die Temperatur betrug 140°C und der Druck 80 bar, es wurde ein Zulauf von 0,6 ml/min. eingestellt. Nach der Hydrierung und Filtration über einen Filter erhielt man TMP mit einer APHA-Farbzahl von 17.

### Beispiel 2

Es wurde TMP der Qualität A hydriert, das eine Farbzahl von 62 APHA und einen TMP-Gehalt von 89,9 % aufwies. Die Hydrierung erfolgte in Analogie zu Beispiel 1 in einem Rohrreaktor in Sumpffahrweise an 100 ml des Katalysators A. Die Temperatur betrug ebenfalls 140°C. Der Druck wurde auf 20 bar, der Zulauf auf 0,3 ml/min. eingestellt. Nach der Hydrierung und Filtration über einen Filter erhielt man ein TMP mit einer APHA-Farbzahl von 23.

### Beispiel 3

Die Hydrierung wurde analog Beispiel 2 durchgeführt. Es wurde TMP der Qualität B hydriert, das eine Farbzahl von 18 APHA und einen TMP-Gehalt von 89,9 % aufwies. Der Druck wurde auf 60 bar, der Zulauf auf 0,3 ml/min. eingestellt. Nach der Hydrierung und Filtration über einen Filter erhielt man TMP mit einer APHA-Farbzahl von 1.

Um auszuschliessen, dass die Filtration keinen Farbzahlverbessernden Effekt aufweist, wurde das Ausgangsmaterial über den Filter gegeben und die Farbzahl anschließend gemessen. Sie war mit der Farbzahl des Eduktes identisch.

## Patentansprüche

1. Verfahren zur Farbzahlverbesserung von mehrwertigen Alkoholen, insbesondere Trimethylolpropan, durch katalytische Hydrierung, wobei der in der Hydrierung eingesetzte mehrwertige Alkohol nach seiner Herstellung durch Destillation gereinigt wurde, **dadurch gekennzeichnet, dass** in Gegenwart eines makroporösen, geträgerten Heterogenkatalysator, der als Aktivmetall mindestens ein Metall der VII. bis X. Nebengruppe des Periodensystems enthält, hydriert wird.

2. Verfahren nach Anspruch 1, **dadurch gekennzeichnet, daß** der in der Hydrierung eingesetzte mehrwertige Alkohol einen Produktgehalt > 95 %, vorzugsweise, im Fall von Trimethylolpropan, > 98 % aufweist.

3. Verfahren nach Anspruch 1 oder 2, **dadurch gekennzeichnet, daß** der makroporösen Heterogenkatalysators als Aktivmetalle Palladium, Ruthenium, Rhenium, Nickel, Eisen und/oder Cobalt oder ein Gemisch aus zwei oder mehr dieser Aktivmetalle enthält.

4. Verfahren nach Anspruch 3, **dadurch gekennzeichnet**, die Makroporosität des Heterogenkatalysators bei mehr als 10 Vol.-% liegt.

5. Verfahren nach Anspruch 4, **dadurch gekennzeichnet, dass** man Aluminiumoxid und/oder Zirkoniumdioxid als Trägermaterial verwendet.

6. Verfahren nach Anspruch 5, **dadurch gekennzeichnet, daß** der Heterogenkatalysator eine Metalloberfläche von 0,01 bis 10 m²/g aufweist.

7. Verfahren nach Anspruch 6, **dadurch gekennzeichnet, dass** der Heterogenkatalysator eine BET-Oberfläche von 0.1 bis 300 m²/g aufweist.

8. Verfahren nach Anspruch 7, **dadurch gekennzeichnet, dass** der Heterogenkatalysator einen Gehalt an Aktivmetall von 0.01 bis 10 Gew.-% aufweisc.

9. Verfahren nach einem der Ansprüche 1 bis 8, **dadurch gekennzeichnet, daß** die Hydrierung bei Temperaturen von 20 bis 300°C, vorzugsweise 100 bis 180°C, durchgeführt wird.

10. Verfahren nach einem der Ansprüche 1 bis 9, **dadurch gekennzeichnet, dass** die Hydrierung bei Drücken von 1 bis 350 bar, vorzugsweise 1 bis 100 bar, meist bevorzugt 1 bis 50 bar, durchgeführt wird.

11. Verfahren nach einem der Ansprüche 1 bis 10, **dadurch gekennzeichnet, dass** die Verweilzeiten des TMP im Reaktor von 5 min bis 20 Stunden, vorzugsweise 10 min bis 8 Stunden, betragen.

12. Verfahren nach einem der Ansprüche 1 bis 11, **dadurch gekennzeichnet, dass** die Hydrierung diskontinuierlich, vorzugsweise im Rührkessel, oder kontinuierlich, vorzugsweise in Rohrreaktoren in Sumpf- oder Rieselfahrweise, betrieben wird.

13. Verfahren nach einem der Ansprüche 1 bis 9, **dadurch gekennzeichnet, daß** die Hydrierung des Trimethylolpropans ohne Zusatz von Lösungsmittel oder unter Zusatz von 5 bis 95 Gew.-% eines Lösungsmittels durchgeführt wird.

## Claims

1. A process for improving the color number of polyhydric alcohols, especially trimethylolpropane, by catalytic hydrogenation, the polyhydric alcohol used in the hydrogenation having been purified by distillation following its preparation, wherein the hydrogenation is carried out in the presence of a macroporous supported heterogeneous catalyst comprising, as the active metal, at least one metal of subgroups VII to X of the Periodic Table.

2. The process according to claim 1 wherein the polyhydric alcohol used in the hydrogenation has a product content of >95% or, in the case of trimethylolpropane, preferably >98%.

3. The process according to claim 1 or 2 wherein the macroporous heterogeneous catalyst comprises, as active metals, palladium, ruthenium, rhenium, nickel, iron and/or cobalt or a mixture of two or more of these active metals.

4. The process according to claim 3 wherein the macroporosity of the heterogeneous catalyst is greater than 10% by volume.

5. The process according to claim 4 wherein aluminum oxide and/or zirconium dioxide are used as the support material.

6. The process according to claim 5 wherein the heterogeneous catalyst has a surface area of metal of 0.01 to 10 m²/g.

7. The process according to claim 6 wherein the heterogeneous catalyst has a BET surface area of 0.1 to 300 m²/g.

8. The process according to claim 7 wherein the heterogeneous catalyst has an active metal content of 0.01 to 10% by weight.

9. The process according to any of claims 1 to 8 wherein the hydrogenation is carried out at temperatures of 20 to 300°C, preferably of 100 to 180°C.

10. The process according to any of claims 1 to 9 wherein the hydrogenation is carried out at pressures of 1 to 350 bar, preferably of 1 to 100 bar and particularly preferably of 1 to 50 bar.

11. The process according to any of claims 1 to 10 wherein the residence times of the TMP in the reactor are from 5 min to 20 hours, preferably 10 min to 8 hours.

12. The process according to any of claims 1 to 11 wherein the hydrogenation is carried out batchwise, preferably in a stirred tank, or continuously, preferably in tubular reactors by the liquid phase or trickle method.

13. The process according to any of claims 1 to 9 wherein the hydrogenation of trimethylolpropane is carried out without the addition of a solvent or with the addition of 5 to 95% by weight of a solvent.

## Revendications

1. Procédé pour améliorer l'indice de couleur d'alcools polyvalents, en particulier de triméthylolpropane, par hydrogénation catalytique, dans lequel l'alcool polyvalent mis en oeuvre dans l'hydrogénation a été purifié par distillation après sa préparation, **caractérisé en ce qu'**on hydrogène en présence d'un catalyseur hétérogène macroporeux sur support qui, comme métal actif, contient au moins un métal du groupe secondaire VII à X du système périodique.

2. Procédé suivant la revendication 1, **caractérisé en ce que** l'alcool polyvalent mis en oeuvre dans l'hydrogénation présente une teneur en produit >95 %, de préférence, dans le cas de triméthylolpropane, >98 %.

3. Procédé suivant la revendication 1 ou 2, **caractérisé en ce que** le catalyseur hétérogène macroporeux contient, comme métaux actifs, du palladium, du ruthénium, du rhénium, du nickel, du fer et/ou du cobalt ou un mélange de deux ou de plusieurs de ces métaux actifs.

4. Procédé suivant la revendication 3, **caractérisé en ce que** la macroporosité du catalyseur hétérogène est de plus de 10 % en volume.

5. Procédé suivant la revendication 4, **caractérisé en ce qu'**on utilise de l'oxyde d'aluminium et/ou du dioxyde de zirconium comme matière de support.

6. Procédé suivant la revendication 5, **caractérisé en ce que** le catalyseur hétérogène présente une surface de métal de 0,01 à 10 m²/g.

7. Procédé suivant la revendication 6, **caractérisé en ce que** le catalyseur hétérogène présente une surface spécifique BET de 0,1 à 300 m²/g.

8. Procédé suivant la revendication 7, **caractérisé en ce que** le catalyseur hétérogène présente une teneur en métal actif de 0,01 à 10 % en poids.

9. Procédé suivant l'une des revendications 1 à 8, **caractérisé en ce que** l'hydrogénation est effectuée à des températures de 20 à 300°C, de préférence de 100 à 180°C.

10. Procédé suivant l'une des revendications 1 à 9, **caractérisé en ce que** l'hydrogénation est effectuée à des pressions de 1 à 350 bars, de préférence de 1 à 100 bars, le plus préférentiellement de 1 à 50 bars.

11. Procédé suivant l'une des revendications 1 à 10, **caractérisé en ce que** les temps de séjour du TMP dans le réacteur sont de 5 minutes à 20 heures, de préférence de 10 minutes à 8 heures.

12. Procédé suivant l'une des revendications 1 à 11, **caractérisé en ce que** l'hydrogénation est mise en oeuvre de manière discontinue, de préférence dans une cuve d'agitation, ou de manière continue, de préférence dans des réacteurs tubulaires dans une mise en service en phase liquide ou avec ruissellement.

13. Procédé suivant l'une des revendications 1 à 9, **caractérisé en ce que** l'hydrogénation du triméthylolpropane est effectuée sans addition de solvant ou avec addition de 5 à 95 % en poids d'un solvant.
